# DEMANDE DE BREVET EUROPEEN

(11) **EP 1 125 556 A1**
(43) Date de publication de la demande: **22.08.2001**
(21) Numéro de dépôt: 00870026.2
(22) Date de dépôt: 18.02.2000
(51) Int. Cl.: A61B 17/42

(54) **Manipulateur uterin**

(71) Demandeur: Medsys S.A., 5030 Gembloux (BE)
(72) Inventeur: Donnez, Jacques, B-1150 Bruxelles (BE); Andre, Jacques, B-1410 Waterloo (BE); Moreels, Xavier, B-5030 Gembloux (BE)
(74) Mandataire: Van Malderen, Joelle

(57) **Abrégé**

La présente invention se rapporte à un manipulateur utérin comprenant au moins (i) un manche présentant un axe longitudinal (10), (ii) un bras 2 présentant également un axe longitudinal (20) avec une extrémité distale (21) le reliant au manche (1) et une extrémité proximale (22), et (iii) une tête (3) présentant un axe longitudinal (30) et fixée à l'extrémité proximale (22) du bras (2), ledit bras (2) étant de forme et de longueur suffisantes pour permettre l'introduction de la tête (3) dudit bras dans la cavité vaginale, caractérisé en ce que ladite tête (3) est munie d'un dispositif sectionnant (4) disposé sur un support (5) permettant de sectionner la paroi vaginale, ce dispositif sectionnant (4) étant disposé à une certaine distance de l'axe longitudinal (30) de la tête (3) et étant susceptible d'effectuer un mouvement de rotation, de préférence de 360 degrés, autour dudit axe de longitudinal (30).

## Description

### Objet de l'invention

La présente invention se rapporte à un manipulateur utérin destiné à être utilisé en hystérectomie vaginale assistée par laparoscopie ou en hystérectomie laparoscopique totale.

### Etat de la technique

Les manipulateurs utérins ont essentiellement pour fonction d'aider le chirurgien à visualiser par laparoscopie la cavité péritonéale et l'utérus, lors d'un examen de contrôle, de diagnostic ou lors d'une opération chirurgicale, en particulier une hystérectomie.

On connaît différents types de manipulateurs utérins qui sont constitués essentiellement d'un bras muni d'une tête destinée à être introduite par voie vaginale à l'intérieur de l'utérus.

Que ce soit lors d'un simple contrôle ou d'une opération chirurgicale au niveau de l'utérus, le chirurgien se trouve confronté à un certain nombre de problèmes : limitation inhérente de la visibilité, identification anatomique, manipulation des organes.

Dans le cas particulier d'une opération chirurgicale, une difficulté supplémentaire vient du fait que l'incision doit être précise et doit éviter d'endommager les tissus environnants.

Classiquement, lorsque l'on procède par laparoscopie pour visualiser la cavité péritonéale et l'utérus, on pratique une petite incision dans l'abdomen pour y introduire un laparoscope tandis que l'on fait entrer par ailleurs le manipulateur dans le vagin. On insuffle alors un gaz, par exemple du CO₂. Quand il s'agit d'opérer, l'acte, chirurgical est alors pratiqué à l'aide d'instruments introduits par l'intermédiaire de petites incisions pratiquées au niveau de l'aine.

Un certain nombre de manipulateurs ont été développés pour aider le chirurgien à visualiser/intervenir dans l'utérus et lui faciliter son travail de chirurgie ou d'examen.

Certains manipulateurs possèdent un premier ballonnet qui est positionné dans l'utérus et dont le gonflement permet de fixer le manipulateur à la paroi utérine et de mobiliser l'utérus. Lorsque ces manipulateurs sont munis de moyens permettant l'introduction de liquides tels que des liquides de contraste dans la cavité utérine en vue d'effectuer un diagnostic, ce premier ballonnet permet en outre d'éviter le reflux de ces liquides par le col utérin. Eventuellement, un deuxième ballonnet positionné au niveau vaginal peut servir d'élément obturateur pour empêcher les pertes de gaz lorsque la paroi vaginale est incisée.

Le brevet US-A-5840077 est un exemple de telles réalisations qui peut être utilisée pour pratiquer une hystérectomie par laparoscopie.

La tête d'un bras manipulable possède un extenseur vaginal qui est bloqué à l'entrée de l'utérus au niveau du col de l'utérus et un embout qui pénètre dans l'utérus. L'extenseur vaginal est de préférence en plastique mais pourrait être aussi en acier inoxydable ou en composite de gaze rigide. Il peut prendre différentes formes.

L'extenseur vaginal peut prendre la forme d'une bague avec dans sa partie distale un rebord circulaire biseauté qui sert de délimitation anatomique et de butoir lors de l'incision, laquelle s'en trouve simplifiée. Des fenêtres pratiquées dans son épaisseur facilitent la visualisation.

Mais l'extenseur vaginal peut également prendre la forme d'un doigt ou d'une demi-cupule.

Le bras du manipulateur quant à lui est articulé et peut pivoter autour de son axe, ce qui permet de positionner correctement l'utérus et contribue aussi à faciliter l'opération.

Néanmoins, les manipulateurs disponibles actuellement présentent un certain nombre d'inconvénients.

Habituellement, les éléments disposés sur la tête de tels manipulateurs sont des consommables qui peuvent être parfois relativement onéreux.

En outre, le fait d'utiliser un élément obturateur sous la forme d'un ballonnet ou d'un cône opaque empêche une bonne visibilité de l'espace vaginal lors du positionnement initial du manipulateur.

Enfin, la section de la paroi vaginale par laparoscopie est difficile à réaliser.

### Buts de l'invention

La présente invention vise à remédier à ces inconvénients.

La présente invention vise en particulier à proposer de simplifier la technique de l'hystérectomie vaginale assistée par laparoscopie en assurant une bonne observation des organes et une identification anatomique fiable.

La présente invention vise également à proposer un manipulateur qui permettrait d'inciser et de découper la paroi vaginale directement par voie vaginale, ce qui augmenterait la sécurité et la rapidité de cette étape et donc de l'intervention chirurgicale dans son ensemble.

La présente invention vise également à proposer un dispositif qui permet d'obturer correctement la cavité utérine tout en permettant une bonne visualisation du col utérin.

### Principaux éléments caractéristiques de l'invention

La présente invention se rapporte à un manipulateur utérin comprenant au moins (i) un manche présentant un axe longitudinal, (ii) un bras présentant également un axe longitudinal avec une extrémité distale le reliant au manche et une extrémité proximale, et (iii) une tête présentant un axe longitudinal et fixée à l'extrémité proximale du bras, ledit bras étant de forme et de longueur suffisantes pour permettre l'introduction de la tête dans la cavité vaginale, caractérisé en ce que ladite tête est munie d'un dispositif sectionnant disposé sur un support permettant de sectionner la paroi vaginale, ce dispositif sectionnant étant disposé à une certaine distance de l'axe longitudinal de la tête et étant susceptible d'effectuer un mouvement de rotation, de préférence de 360 degrés, autour dudit axe longitudinal.

De préférence, l'axe longitudinal de la tête du dispositif et l'axe longitudinal du bras font un angle de façon à adapter l'orientation de la tête à l'anatomie utérine, ledit angle étant de préférence de 25°.

De préférence, le dispositif sectionnant est une électrode bipolaire, unipolaire ou similaire.

Avantageusement, le support du dispositif sectionnant se présente sous la forme d'une ceinture circulaire se déplaçant sur la tête en effectuant un mouvement de rotation autour de l'axe de la tête.

De préférence, le mouvement de rotation de la ceinture est commandé par des moyens de commande à partir du manche.

De préférence, lesdits moyens de commande comprennent un tube présentant un axe longitudinal et situé essentiellement à l'intérieur du manche, ledit manche et ledit tube étant concentriques et le tube étant susceptible d'effectuer un mouvement de rotation autour de son axe longitudinal, ce mouvement générant un mouvement de rotation de ladite ceinture par l'intermédiaire de moyens de transmission.

De préférence, lesdits moyens de transmission comprennent une tige avec un axe longitudinal et située essentiellement à l'intérieur du bras, ladite tige étant susceptible d'effectuer un mouvement de rotation autour de son axe, permettant ainsi de transmettre le mouvement de rotation du tube à la ceinture à la tête.

De préférence, lesdits moyens de transmission comprennent en outre un croisillon situé à l'extrémité proximale du bras.

De plus, le manipulateur comprend en outre des moyens de commande pour commander le positionnement du dispositif sectionnant selon deux positions le long de l'axe longitudinal de la tête, une position dite basse et une position dite haute, le dispositif sectionnant ne pouvant fonctionner (subir de rotation) que lorsqu'il est dans la position haute (en position de travail).

De préférence, lesdits moyens de commande comprennent un levier se déplaçant entre une position haute et une position basse et une couronne, ladite couronne étant solidaire à la fois de l'axe du bras et de l'axe du tube et ce levier prenant appui sur ladite couronne afin de pouvoir se déplacer en rotation.

De préférence, le manipulateur comprend en outre des moyens de sécurité qui permettent de bloquer le dispositif sectionnant dans sa position haute.

De préférence, lesdits moyens de sécurité comprennent un anneau guide capable de bloquer le levier dans la position haute.

De préférence, les moyens de commande présentent un guide en vue d'éviter la rotation du dispositif sectionnant en position de repos.

Avantageusement, la tête se présente sous la forme d'une cupule, qui peut être réalisée en un matériau transparent.

Cette cupule présente des dimensions adéquates permettant d'agir comme obturateur au niveau du col de l'utérus.

Selon une forme particulière de l'invention, la cupule est munie de dents afin d'éviter tout déplacement ou glissement latéral après sa pose.

La présence des dents permet également avantageusement de rendre le mouvement de torsion de l'utérus possible.

La présente invention se rapporte également à un manipulateur utérin constitué essentiellement d'un bras présentant une extrémité distale et une extrémité proximale et muni d'une tête présentant un axe longitudinal, ledit bras étant de forme et de longueur suffisantes pour permettre l'introduction de la tête dudit bras dans la cavité vaginale, caractérisé en ce que ladite tête est munie d'une cupule transparente.

De préférence, la cupule présente des dimensions adéquates permettant d'agir comme obturateur au niveau du col de l'utérus.

Avantageusement, ladite cupule est dentée.

### Brève description des dessins

La figure 1 représente une vue en coupe longitudinale du manipulateur selon la présente invention.

La figure 2 est une vue en coupe longitudinale de la tête du manipulateur avec l'électrode et la ceinture circulaire selon une forme préférée d'exécution de la présente invention.

La figure 3 est une vue en coupe longitudinale de la cupule correspondant à la tête du manipulateur selon une forme préférée d'exécution de la présente invention.

La figure 4 représente une vue du dessus de la cupule correspondant à la tête du manipulateur selon une forme préférée d'exécution de la présente invention.

La figure 5 représente une vue en coupe longitudinale du manche avec le levier et la couronne selon une forme préférée d'exécution de la présente invention.

La figure 6 représente une vue du dessus du manche avec le levier et la couronne selon une forme préférée d'exécution de la présente invention.

### Description d'une forme d'exécution préférée de l'invention

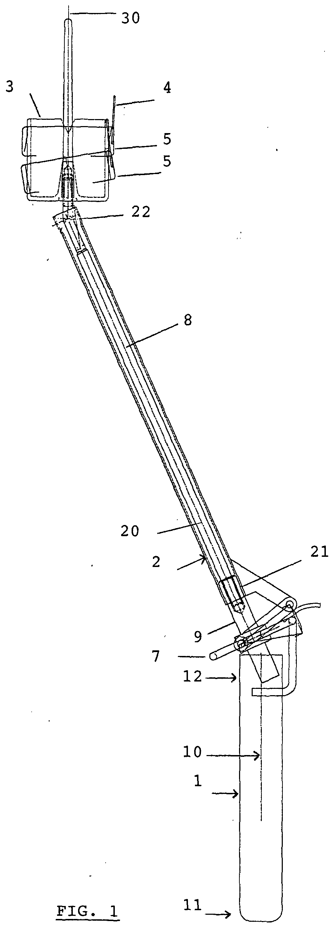

Une vue générale en coupe longitudinale d'une forme préférée d'exécution du manipulateur selon la présente invention est représentée sur la figure 1.

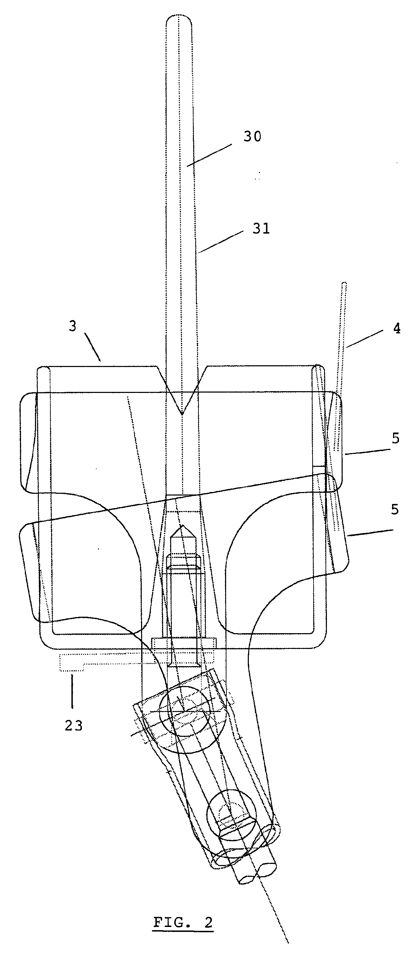

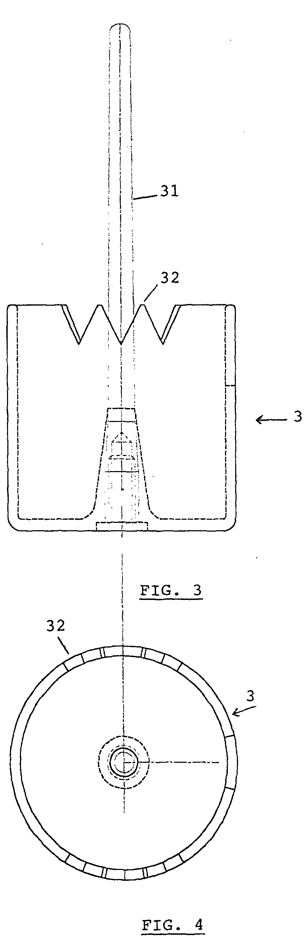

Le manipulateur comprend un manche 1 qui présente un axe longitudinal 10, une extrémité distale 11 et une extrémité proximale 12 au niveau de laquelle est fixé un bras 2. Le bras 2 présente lui aussi un axe longitudinal 20, une extrémité distale 21 et une extrémité proximale 22 au niveau de laquelle est fixée une tête qui se présente sous la forme d'une cupule 3 avec un axe longitudinal 30 et telle que représentée aux figures 2 à 4. Avantageusement, la cupule 3 est réalisée en un matériau transparent pour voir au travers et faciliter ainsi l'acte chirurgical. Cette cupule 3 présente des dimensions adéquates pour faire fonction d'obturateur au niveau du col de l'utérus. La cupule 3 présente en son centre un orientateur 31 capable d'imposer un mouvement de torsion à l'utérus. La cupule 3 est munie de dents 32 afin d'éviter tout déplacement ou glissement latéral du manipulateur lors de son utilisation. Les dents 32 contribuent également, avec l'orientateur 31, à rendre le mouvement de torsion de l'utérus possible.

Le bras 2 est de longueur et de forme suffisantes pour permettre l'introduction de la cupule 3 dans la cavité vaginale. En outre, l'axe longitudinal 20 du bras 2 fait avec un axe longitudinal 30 de la cupule 3 un angle bien défini, de préférence 25°, de façon à adapter l'orientation de la cupule à l'anatomie utérine.

A l'intérieur du bras 2 se trouve une tige 8, le bras 2 et la tige 8 étant concentriques et l'axe longitudinal de la tige 8 étant donc confondu avec l'axe longitudinal 20 du bras 2.

C'est à l'intérieur de la cupule 3 qu'est situé un dispositif sectionnant permettant de réaliser une hystérectomie. Ce dispositif sectionnant correspond à une électrode 4 qui peut être bipolaire, unipolaire ou similaire. Cette électrode 4 est située à une certaine distance de l'axe longitudinal 30 de la cupule 3 et est susceptible d'effectuer un mouvement de rotation, de préférence de 360 degrés, autour dudit axe longitudinal 30. L'électrode 4 est par ailleurs disposée sur un support correspondant à une ceinture circulaire 5 capable de se déplacer par rapport à la cupule en effectuant un mouvement de rotation autour de l'axe longitudinal 30 de la cupule 3.

Des moyens de commande du mouvement de rotation de la ceinture circulaire 5 et donc celui de l'électrode 4 se trouvent au niveau du manipulateur. Ces moyens de commande constituent également des moyens de commande de la position de la ceinture circulaire 5 et donc de l'électrode 4 le long de l'axe longitudinal 30 de la cupule 3 suivant deux positions, une position basse A et une position haute B.

Un premier moyen de commande est constitué par une tige (non représentée) située au niveau du manche 1 et reliée à un connecteur 9.

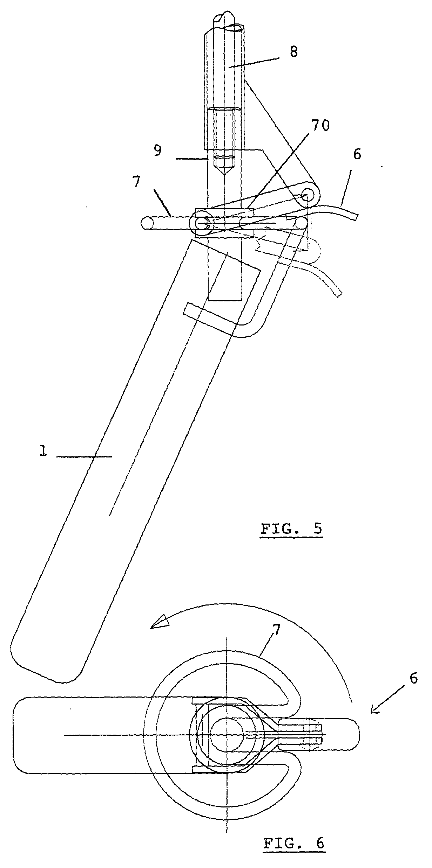

Un deuxième moyen de commande de la position de la ceinture circulaire 5 et donc de l'électrode 4 selon une position haute B ou basse A est constitué par un levier 6 capable de prendre appui sur une couronne 7, tel que représenté à la figure 5, et solidaire à la fois du bras 2 et de la tige 8 située à l'intérieur de ce bras 2. Le levier peut adopter deux positions, une position basse A et une position B. La conformation de la couronne 7 et du levier 6 est telle que le levier 6 prend appui sur la couronne 7 tout au long de son parcours circulaire en position haute B mais pas en position basse A, comme le montre la figure 6.

Des moyens de transmission du mouvement de rotation ainsi que de la position haute A ou basse B de la ceinture circulaire 5 et donc de l'électrode 4 depuis les moyens de commande jusqu'à ladite ceinture circulaire 5 et l'électrode 4 sont également prévus au niveau du manipulateur.

Ces moyens de transmission comprennent la tige 8 du bras 2 qui est capable de tourner autour de l'axe longitudinal 20 du bras 2 et de se déplacer le long dudit axe 20.

Ces moyens de transmission comprennent également un croisillon 23 situé à l'extrémité proximale 22 du bras 2.

Des moyens de sécurité permettent de sécuriser le manipulateur. Ces moyens de sécurité correspondent ici à un anneau guide 70 avec une encoche dans laquelle vient s'emboîter le levier 6 en position haute B permettant ainsi de le bloquer dans cette position et d'éviter la rotation de la ceinture circulaire 5 et donc de l'électrode 4.

En position de repos, c'est-à-dire lorsque l'on n'utilise pas l'électrode 4, le manipulateur est dans une configuration où l'électrode 4 et la ceinture circulaire 5 mais aussi le levier 6 sont en position basse. Dans cette position, l'électrode 4 et la ceinture circulaire 5 sont situées à l'intérieur de la cupule 3.

Pour utiliser l'électrode 4, il faut suivre les étapes suivantes :
- dans un premier temps, on pousse le tube situé au niveau du manche 1, ce qui a pour effet d'actionner le levier 6 et de le faire passer de sa position basse A à sa position haute B. Dans sa position haute B, le levier 6 vient alors s'emboîter dans l'anneau guide 70, et le bloque ainsi dans cette position. Le levier 6 prend alors appui sur la couronne 7. Le déplacement du levier 6 de sa position basse A à sa position haute B a pour effet d'abord de déplacer la tige 8 située à l'intérieur du bras 2 et ensuite de déplacer la ceinture circulaire 5 et l'électrode 4 de la position basse A à la position haute B. Dans cette nouvelle position, l'électrode 4 fait saillie en dehors de la cupule 3 et est ainsi prête a subir un mouvement de rotation ;
- dans un deuxième temps, on fait subir au levier 6 un mouvement de rotation qui est transmis à la ceinture circulaire 5 et donc à l'électrode 4 qui peut alors être utilisée pour l'hystérectomie.

## Revendications

1. Manipulateur utérin comprenant au moins (i) un manche présentant un axe longitudinal (10), (ii) un bras 2 présentant également un axe longitudinal (20) avec une extrémité distale (21) le reliant au manche (1) et une extrémité proximale (22), et (iii) une tête (3) présentant un axe longitudinal (30) et fixée à l'extrémité proximale (22) du bras (2), ledit bras (2) étant de forme et de longueur suffisantes pour permettre l'introduction de la tête (3) dudit bras dans la cavité vaginale, caractérisé en ce que ladite tête (3) est munie d'un dispositif sectionnant (4) disposé sur un support (5) permettant de sectionner la paroi vaginale, ce dispositif sectionnant (4) étant disposé à une certaine distance de l'axe longitudinal (30) de la tête (3) et étant susceptible d'effectuer un mouvement de rotation, de préférence de 360 degrés, autour dudit axe de longitudinal (30).

2. Manipulateur utérin selon la revendication 1, caractérisé en ce que l'axe longitudinal (30) de la tête (3) du dispositif et l'axe longitudinal (20) du bras (2) font un angle de façon à adapter l'orientation de la tête à l'anatomie utérine, ledit angle étant de préférence de 25°.

3. Manipulateur utérin selon la revendication 1, caractérisé en ce que le dispositif sectionnant est une électrode (4) bipolaire, unipolaire ou similaire.

4. Manipulateur utérin selon la revendication 1, caractérisé en ce que le support du dispositif sectionnant se présente sous la forme d'une ceinture circulaire (5) se déplaçant sur la tête (3) en effectuant un mouvement de rotation autour de l'axe longitudinal (30) de la tête (3).

5. Manipulateur utérin selon la revendication 4, caractérisé en ce que le mouvement de rotation de la ceinture (5) est commandé par des moyens de commande à partir du manche.

6. Manipulateur utérin selon la revendication 5, caractérisé en ce que lesdits moyens de commande comprennent un tube présentant un axe longitudinal et situé essentiellement à l'intérieur du manche (1), ledit manche (1) et ledit tube étant concentriques et le tube étant susceptible d'effectuer un mouvement de rotation autour de son axe longitudinal, ce mouvement générant un mouvement de rotation de ladite ceinture par l'intermédiaire de moyens de transmission.

7. Manipulateur utérin selon la revendication 6, caractérisé en ce que lesdits moyens de transmission comprennent une tige (8) avec un axe longitudinal (20), située essentiellement à l'intérieur du bras (2), ledit bras (2) et ladite tige (8) étant concentriques, ladite tige (8) étant susceptible d'effectuer un mouvement de rotation autour de son axe (20), permettant ainsi de transmettre à la ceinture (5) le mouvement de rotation du tube situé essentiellement à l'intérieur du manche (1).

8. Manipulateur utérin selon la revendication 7, caractérisé en ce que lesdits moyens de transmission comprennent en outre un croisillon (23) situé à l'extrémité proximale (22) du bras (2).

9. Manipulateur utérin selon l'une des revendications précédentes, caractérisé en ce que le manipulateur comprend en outre des moyens de commande pour commander le positionnement du dispositif sectionnant (4) selon deux positions le long de l'axe longitudinal de la tête, une position dite basse A et une position dite haute B, le dispositif sectionnant ne pouvant subir de rotation que lorsqu'il est dans la position haute B.

10. Manipulateur utérin selon la revendication 9, caractérisé en ce que lesdits moyens de commande comprennent un levier 6 se déplaçant entre une position basse A et une position basse B et une couronne 7, ladite couronne 7 étant solidaire à la fois du bras (2) et de la tige (8) et ce levier 6 prenant appui sur ladite couronne afin de pouvoir se déplacer en rotation.

11. Manipulateur utérin selon la revendication 10, caractérisé en ce que le manipulateur comprend en outre des moyens de sécurité qui permettent de bloquer le dispositif sectionnant (4) dans sa position haute (B).

12. Manipulateur utérin selon la revendication 11, caractérisé en ce que lesdits moyens de sécurité comprennent un anneau guide capable de bloquer le levier dans la position haute (B).

13. Manipulateur utérin selon la revendication 6, caractérisé en ce que les moyens de commande présentent un anneau guide (70) en vue d'éviter la rotation du dispositif sectionnant en position basse (A).

14. Manipulateur utérin selon l'une des revendications précédentes, caractérisé en ce que la tête se présente sous la forme d'une cupule (3).

15. Manipulateur utérin selon la revendication 14, caractérisé en ce que ladite cupule (3) est réalisée en un matériau transparent.

16. Manipulateur utérin selon la revendication 14 ou 15, caractérisé en ce ladite cupule (3) présente des dimensions adéquates permettant d'agir comme obturateur au niveau du col de l'utérus.

17. Manipulateur utérin selon l'une des revendications 14 à 16, caractérisé en ce que la cupule (3) est munie de dents.

18. Manipulateur utérin, constitué essentiellement d'un bras (2) présentant une extrémité distale (21) et une extrémité proximale (22) et muni d'une tête présentant un axe longitudinal (30), ledit bras (2) étant de forme et de longueur suffisantes pour permettre l'introduction de la tête dudit bras (2) dans la cavité vaginale, caractérisé en ce que ladite tête se présente sous la forme d'une cupule transparente (3).

19. Manipulateur utérin selon la revendication 18, caractérisé en ce que la cupule (3) présente des dimensions adéquates permettant d'agir comme obturateur au niveau du col de l'utérus.

20. Manipulateur utérin selon la revendication 18 ou 19, caractérisé en ce que ladite cupule (3) est dentée.
